# EUROPEAN PATENT APPLICATION

(11) **EP 4 495 143 A1**
(43) Date of publication of application: **22.01.2025**
(21) Application number: 23769872.5
(22) Date of filing: 16.03.2023
(51) Int. Cl.: C07K 19/00, A61K 38/28, A61K 38/16, A61P 3/10

(54) **HUMAN INSULIN ANALOGUE, AND FUSION PROTEIN THEREOF AND MEDICAL USE THEREOF**

(30) Priority: 16.03.2022 CN 202210259967
(71) Applicant: Beijing Tuo Jie Biopharmaceutical Co. Ltd., Beijing 102206 (CN)
(72) Inventor: WU, Fangzhou, Beijing 102206 (CN); JIANG, Peng, Beijing 102206 (CN); SHEN, Chenxi, Beijing 102206 (CN); DU, Yanping, Beijing 102206 (CN); LIU, Renzhi, Beijing 102206 (CN); LI, Yuanyuan, Beijing 102206 (CN); HUANG, Xuchao, Beijing 102206 (CN); HE, Xugang, Beijing 102206 (CN); WANG, Lei, Beijing 102206 (CN)
(74) Representative: Dragotti & Associati S.R.L.
(86) International application number: PCT/CN2023/081885
(87) International publication number: WO 2023/174370

(57) **Abstract**

A human insulin analogue, and a fusion protein thereof and the medical use thereof. The human insulin analogue-Fc fusion protein has ultra-long in-vivo pharmacokinetic properties and can be used for treating metabolic diseases (such as type-I diabetes and type-II diabetes).

## Description

The present application claims priority to Chinese Patent Application No. 202210259967.6 filed on March 16, 2022.

### TECHNICAL FIELD

The present disclosure relates to the field of biological medicine and particularly to a class of human insulin analogs and fusion proteins thereof, and use of the fusion proteins in the treatment of metabolic diseases (e.g., diabetes).

### BACKGROUND

Diabetes is a chronic metabolic disease in which the metabolism of glucose, proteins and lipids in a human body is disordered due to insufficient insulin secretion in the body. It is mainly classified into insulin-dependent diabetes (type I diabetes) and non-insulin-dependent diabetes (type II diabetes). Globally, 90-95% of diabetes patients have type II diabetes, which is caused by impaired pancreatic β cell function and long-term insulin resistance, and is primarily characterized by a lack of insulin levels in the body, decreased insulin sensitivity, and high blood glucose concentrations in the plasma. Studies have shown that type II diabetes is associated with a variety of high-risk complications, which can lead to cardiovascular disease, kidney failure, blindness, amputation, and many other complications.

Approved diabetes drugs on the market mainly include chemically synthesized small-molecule oral hypoglycemic drugs such as biguanides, sulfonyl compounds, insulin sensitizers and α-glycosides, as well as injectable peptide hypoglycemic drugs such as glucagon-like peptide-1 (GLP-1) analogs. For all type I diabetes and some end-stage type II diabetes, only insulin injection-related drugs can better control the blood glucose levels.

Currently, approved insulin analog products on the market are mainly divided into two categories: rapid-acting insulin and long-acting basal insulin. Rapid-acting insulins that are already on the market include insulin lispro, insulin aspart, and the like. The rapid-acting insulins are primarily administered to patients via subcutaneous injection before meals. They have modified insulin molecular structures to inhibit the formation of natural insulin hexamers, thus having pharmacokinetic characteristics of rapid onset and quick clearance after being administered via subcutaneous injection. Insulin lispro achieves its inhibitory effect on the formation of insulin hexamers by switching the amino acids at positions 28 and 29 at the terminus of the natural insulin B-chain, and insulin aspart inhibits the formation of hexamers by replacing proline with aspartic acid at position 28 on the insulin B-chain and through charge repulsion interactions. Approved long-acting basal insulins on the market include insulin glargine, insulin detemir, and insulin degludec. Insulin glargine has positively charged arginine introduced into the insulin molecular skeleton to raise the molecular isoelectric point to neutrality, so that after being administered via subcutaneous injection, the insulin analog forms a precipitate at the site of administration, and then the precipitate slowly dissolves to provide a sustained release, thereby achieving significantly extended *in-vivo* pharmacokinetic properties. Insulin detemir and insulin degludec have long-chain fatty acid modifications introduced to the lysine side chain at the terminus of the B-chain, so as to achieve extended *in-vivo* pharmacokinetic properties by forming supramolecular polymers at the site of administration and by reversibly binding to human serum albumin in the plasma. Currently, the frequency of administration of insulin glargine and insulin degludec is once a day, while the frequency of administration of insulin detemir is once or twice a day.

However, the rapid-acting insulin products may cause adverse reactions such as hypoglycemia and severe hypoglycemia symptoms, and patients need to be injected with insulin drugs once or even multiple times a day, leading to inconvenience in use, heavy treatment burden, and decreased quality of life for the patients. The existing long-acting basal insulin products also need to be administered via subcutaneous injection once a day, so the injection frequency is still relatively high. Therefore, ultra-long-acting basal insulin products with lower injection frequency and higher safety are the demand of clinical patients, which can significantly improve patient compliance and convenience. The present disclosure provides such ultra-long-acting basal insulin products.

### SUMMARY

The present disclosure provides a fusion protein of a human insulin analog and an IgG Fc region, a preparation method therefor, and use thereof in the treatment of metabolic diseases (e.g., type I diabetes or type II diabetes).

### Insulin Analog

The present disclosure provides an insulin analog comprising an insulin B-chain analog and an insulin A-chain analog, wherein the insulin B-chain analog comprises the following amino acid sequence:
FVX₁QHLCGX₂HLVEALX₃X₄VCGERGFX₅X₆X₇X₈X₉ (SEQ ID NO: 43), wherein X₁ is selected from the group consisting of N, G, and K; X₂ is selected from the group consisting of S and E; X₃ is selected from the group consisting of Y, H, and E; X₄ is selected from the group consisting of H, R, L, and E; X₅ is selected from the group consisting of F and H; X₆ is selected from the group consisting of G, T, S, H, V, and Y, or is absent; X₇ is selected from the group consisting of G, E, P, K, D, S, and H, or is absent; X₈ is selected from the group consisting of G, E, K, P, Q, D, and H, or is absent; and X₉ is selected from the group consisting of G, T, S, E, K, and A, or is absent;
the insulin A-chain analog comprises the following amino acid sequence: GIVEQCCZ₁SICSLZ₂QLENYCZ₃Z₄ (SEQ ID NO: 44), wherein Z₁ is selected from the group consisting of T, H, and E, Z₂ is selected from the group consisting of Y, D, S, and
E, and Z₃ is selected from the group consisting of G and N; Z₄ is any natural amino acid or is absent, and when Z₃ is N, Z₄ is not G or N.

The present disclosure provides an insulin analog comprising an insulin B-chain analog and an insulin A-chain analog, wherein the insulin B-chain analog comprises the following amino acid sequence:
FVX₁QHLCGX₂HLVEALX₃X₄VCGERGFX₅Y (SEQ ID NO: 1), wherein X₁ is selected from the group consisting of N, G, and K; X₂ is selected from the group consisting of S and E; X₃ is selected from the group consisting of Y, H, and E; X₄ is selected from the group consisting of H, R, L, and E; and X₅ is selected from the group consisting of F and H;
the insulin A-chain analog comprises the following amino acid sequence: GIVEQCCZ₁SICSLZ₂QLENYCZ₃ (SEQ ID NO: 2), wherein Z₁ is selected from the group consisting of T, H, and E, Z₂ is selected from the group consisting of Y, D, S, and E, and Z₃ is selected from the group consisting of G and N.

In some embodiments, the insulin B-chain analog comprises (or is) the amino acid sequence set forth in SEQ ID NO: 43 or SEQ ID NO: 1, which has at least 1, at least 2, at least 3, at least 4, or 5 amino acid modifications at X₁, X₂, X₃, X₄, and X₅. In some specific embodiments, X₁ is selected from the group consisting of N and K, X₂ is selected from the group consisting of S and E, X₃ is selected from the group consisting of Y and H, X₄ is selected from L, and/or X₅ is selected from H. In some specific embodiments, X₂ is E, and X₅ is H. In some specific embodiments, X₅ is H. In some specific embodiments, X₃ is H, and X₅ is H. In some specific embodiments, X₁ is K, X₂ is E, and X₅ is H.

In some embodiments, the insulin A-chain analog comprises (or is) the amino acid sequence set forth in SEQ ID NO: 44 or SEQ ID NO: 2, which has at least 1, at least 2, or 3 amino acid modifications at Z₁, Z₂, and Z₃. In some specific embodiments, Z₁ is selected from the group consisting of T, H, and E, Z₂ is selected from E, and Z₃ is selected from the group consisting of G and N. In some specific embodiments, Z₁ is E, Z₂ is E, and Z₃ is G. In some specific embodiments, Z₁ is H, Z₂ is E, and Z₃ is G. In some specific embodiments, Z₂ is E. In some specific embodiments, Z₁ is E, and Z₂ is E. In some embodiments, in the insulin analog, the insulin B-chain analog comprises (or is) the sequence of SEQ ID NO: 43 or SEQ ID NO: 1, and the insulin A-chain analog comprises (or is) the amino acid sequence set forth in SEQ ID NO: 44 or SEQ ID NO: 2. In some specific embodiments, X₁ is selected from the group consisting of N and K, X₂ is selected from the group consisting of S and E, X₃ is selected from the group consisting of Y and H, X₄ is selected from L, and X₅ is selected from H; and Z₁ is selected from the group consisting of T, H, and E, Z₂ is selected from the group consisting of E, and Z₃ is selected from the group consisting of G and N. In some specific embodiments, X₂ is E, X₅ is H, Z₁ is E, Z₂ is E, and Z₃ is G. In some specific embodiments, X₅ is H, Z₁ is H, Z₂ is E, and Z₃ is G. In some specific embodiments, X₃ is H, X₅ is H, and Z₂ is E. In some specific embodiments, X₁ is K, X₂ is E, X₅ is H, Z₁ is E, Z₂ is E, and Z₃ is G. In some specific embodiments, X₂ is E, X₅ is H, Z₁ is E, Z₂ is E, and Z₃ is G. In some specific embodiments, X₂ is E, X₃ is H, Z₁ is E, and Z₂ is E.

In some specific embodiments:
X₁ is N or K, X₂ is E, X₃ is Y, X₄ is L, X₅ is H, Z₁ is E, Z₂ is E, and Z₃ is G;
X₁ is N, X₂ is E, X₃ is Y, X₄ is L, X₅ is H, Z₁ is E, Z₂ is E, and Z₃ is N;
X₁ is N, X₂ is S, X₃ is Y, X₄ is L, X₅ is H, Z₁ is H, Z₂ is E, and Z₃ is G;
X₁ is N, X₂ is S, X₃ is H, X₄ is L, X₅ is H, Z₁ is T, Z₂ is E, and Z₃ is G; or
X₁ is N, X₂ is E, X₃ is Y, X₄ is L, X₅ is H, Z₁ is E, Z₂ is E, and Z₃ is G.

In the above embodiments, X₆ to X₉ or X₇ to X₉ may each be G; or X₆ to X₉ or X₇ to X₉ may be absent.

In the above embodiments, Z₄ may be absent; or when Z₃ is N, Z₄ is an amino acid other than G, N, S, V, L, or P.

In some embodiments, in the insulin analog, the insulin B-chain analog comprises (or is) any one of the following:
FVNQHLCGEHLVEALYLVCGERGFHY (SEQ ID NO: 31);
FVNQHLCGSHLVEALYLVCGERGFHY (SEQ ID NO: 32);
FVNQHLCGSHLVEALHLVCGERGFHY (SEQ ID NO: 33);
FVKQHLCGEHLVEALYLVCGERGFHY (SEQ ID NO: 34);
FVNQHLCGEHLVEALYLVCGERGFHY (SEQ ID NO: 35); and
FVNQHLCGEHLVEALYLVCGERGFHY (SEQ ID NO: 36).

In some embodiments, in the insulin analog, the insulin A-chain analog comprises (or is) any one of the following:
GIVEQCCESICSLEQLENYCG (SEQ ID NO: 37);
GIVEQCCHSICSLEQLENYCG (SEQ ID NO: 38);
GIVEQCCTSICSLEQLENYCN (SEQ ID NO: 39);
GIVEQCCESICSLEQLENYCG (SEQ ID NO: 40);
GIVEQCCESICSLEQLENYCG (SEQ ID NO: 41); and
GIVEQCCESICSLEQLENYCN (SEQ ID NO: 42).

In some embodiments, in the insulin analog, the insulin B-chain analog and the insulin A-chain analog respectively comprise (or are) the amino acid sequences set forth in any one of the following groups: SEQ ID NOs: 31 and 37; SEQ ID NOs: 32 and 38; SEQ ID NOs: 33 and 39; SEQ ID NOs: 34 and 40; SEQ ID NOs: 35 and 41; and SEQ ID NOs: 36 and 42.

In some embodiments, a disulfide bond is present in the insulin analog described above. In some specific embodiments, there are two disulfide bonds for linking the insulin A-chain analog and the insulin B-chain analog at CysA7-CysB7 and CysA20-CysB19; and/or there is an intrachain disulfide bond at CysA6-CysA11 in the insulin A-chain analog.

Illustratively, "CysA7-CysB7" indicates that an interchain disulfide bond is formed between the Cys at position 7 from the N-terminus in the insulin A-chain analog and the Cys at position 7 from the N-terminus in the insulin B-chain analog. "CysA20-CysB19" indicates that an interchain disulfide bond is formed between the Cys at position 20 from the N-terminus in the insulin A-chain analog and the Cys at position 19 from the N-terminus in the insulin B-chain analog.

In some embodiments, the insulin analog according to any one of the above embodiments has insulin receptor agonistic activity.

In some embodiments, the above insulin analog has amino acid modifications at any one or more (e.g., 2, 3, 4, 5, 6, 7, 8, etc.) positions in the insulin A-chain analog and the insulin B-chain analog. In some embodiments, the modification is a chemical group modification to improve the chemical and/or physical stability of the insulin analog, regulate the efficacy of the insulin analog, and/or increase the expression of the insulin analog.

In some embodiments, the modified insulin analog is a glucose-responsive insulin.

In some embodiments, the above insulin analog is modified with a fatty acid chain.

### Fusion Protein

The present disclosure provides a fusion protein of a human insulin analog.

In some embodiments, the fusion protein comprises an insulin analog, wherein
the insulin analog has the following general formula: B₁-L₁-A₁, wherein
B₁ is an insulin B-chain analog comprising the following amino acid sequence:
   FVX₁QHLCGX₂HLVEALX₃X₄VCGERGFX₅X₆X₇X₈X₉ (SEQ ID NO: 43), wherein X₁ is selected from the group consisting of N, G, and K; X₂ is selected from the group consisting of S and E; X₃ is selected from the group consisting of Y, H, and E; X₄ is selected from the group consisting of H, R, L, and E; X₅ is selected from the group consisting of F and H; X₆ is selected from the group consisting of G, T, S, H, V, and Y, or is absent; X₇ is selected from the group consisting of G, E, P, K, D, S, and H, or is absent; X₈ is selected from the group consisting of G, E, K, P, Q, D, and H, or is absent; and X₉ is selected from the group consisting of G, T, S, E, K, and A, or is absent;
A₁ is an insulin A-chain analog comprising the following amino acid sequence: GIVEQCCZ₁SICSLZ₂QLENYCZ₃Z₄ (SEQ ID NO: 44), wherein Z₁ is selected from the group consisting of T, H, and E, Z₂ is selected from the group consisting of Y, D, S, and E, and Z₃ is selected from the group consisting of G and N; Z₄ is any natural amino acid or is absent, and when Z₃ is N, Z₄ is not G or N.
L₁ is a linker.

In some embodiments, the fusion protein comprises an insulin analog, wherein
the insulin analog has the following general formula: B₁-L₁-A₁, wherein
B₁ is an insulin B-chain analog comprising the following amino acid sequence: FVX₁QHLCGX₂HLVEALX₃X₄VCGERGFX₅Y (SEQ ID NO: 1), wherein X₁ is selected from the group consisting of N, G, and K, X₂ is selected from the group consisting of S and E, X₃ is selected from the group consisting of Y, H, and E, X₄ is selected from the group consisting of H, R, L, and E, and X₅ is selected from the group consisting of F and H;
A₁ is an insulin A-chain analog comprising the following amino acid sequence: GIVEQCCZ₁SICSLZ₂QLENYCZ₃ (SEQ ID NO: 2), wherein Z₁ is selected from the group consisting of T, H, and E, Z₂ is selected from the group consisting of Y, D, S, and E, and Z₃ is selected from the group consisting of G and N;
L₁ is a linker.

In some embodiments, B₁ comprises (or is) the amino acid sequence set forth in SEQ ID NO: 43 or SEQ ID NO: 1, which has at least 1, at least 2, at least 3, at least 4, or 5 amino acid modifications at X₁, X₂, X₃, X₄, and X₅. In some specific embodiments, X₁ is selected from the group consisting of N and K, X₂ is selected from the group consisting of S and E, X₃ is selected from the group consisting of Y and H, X₄ is selected from L, and/or X₅ is selected from H. In some specific embodiments, X₂ is E, and X₅ is H. In some specific embodiments, X₅ is H. In some specific embodiments, X₃ is H, and X₅ is H. In some specific embodiments, X₁ is K, X₂ is E, and X₅ is H.

In some embodiments, A₁ comprises (or is) the amino acid sequence set forth in SEQ ID NO: 44 or SEQ ID NO: 2, which has at least 1, at least 2, or 3 amino acid modifications at Z₁, Z₂, and Z₃. In some specific embodiments, Z₁ is selected from the group consisting of T, H, and E, Z₂ is selected from E, and Z₃ is selected from the group consisting of G and N. In some specific embodiments, Z₁ is E, Z₂ is E, and Z₃ is G. In some specific embodiments, Z₁ is H, Z₂ is E, and Z₃ is G. In some specific embodiments, Z₂ is E. In some specific embodiments, Z₁ is E, and Z₂ is E.

In some embodiments, in the insulin analog, B₁ comprises (or is) the sequence of SEQ ID NO: 43 or SEQ ID NO: 1, and A₁ comprises (or is) the amino acid sequence set forth in SEQ ID NO: 44 or SEQ ID NO: 2. In some specific embodiments, X₁ is selected from the group consisting of N and K, X₂ is selected from the group consisting of S and E, X₃ is selected from the group consisting of Y and H, X₄ is selected from L, and X₅ is selected from H; and Z₁ is selected from the group consisting of T, H, and E, Z₂ is selected from the group consisting of E, and Z₃ is selected from the group consisting of G and N. In some specific embodiments, X₂ is E, X₅ is H, Z₁ is E, Z₂ is E, and Z₃ is G. In some specific embodiments, X₅ is H, Z₁ is H, Z₂ is E, and Z₃ is G. In some specific embodiments, X₃ is H, X₅ is H, and Z₂ is E. In some specific embodiments, X₁ is K, X₂ is E, X₅ is H, Z₁ is E, Z₂ is E, and Z₃ is G. In some specific embodiments, X₂ is E, X₅ is H, Z₁ is E, Z₂ is E, and Z₃ is G. In some specific embodiments, X₂ is E, X₅ is H, Z₁ is E, and Z₂ is E.

In some specific embodiments, X₁ is N or K, X₂ is E, X₃ is Y, X₄ is L, X₃ is H, Z₁ is E, Z₂ is E, and Z₃ is G;
X₁ is N, X₂ is E, X₃ is Y, X₄ is L, X₅ is H, Z₁ is E, Z₂ is E, and Z₃ is N;
X₁ is N, X₂ is S, X₃ is Y, X₄ is L, X₃ is H, Z₁ is H, Z₂ is E, and Z₃ is G;
X₁ is N, X₂ is S, X₃ is H, X₄ is L, X₅ is H, Z₁ is T, Z₂ is E, and Z₃ is G; or
X₁ is N, X₂ is E, X₃ is Y, X₄ is L, X₅ is H, Z₁ is E, Z₂ is E, and Z₃ is G.

In the above embodiments, X₆ to X₉ or X₇ to X₉ may each be G; or X₆ to X₉ or X₇ to X₉ may be absent.

In the above embodiments, Z₄ may be absent; or when Z₃ is N, Z₄ is an amino acid other than G, N, S, V, L, or P.

In some embodiments, in the insulin analog, B₁ comprises (or is) the amino acid sequence of any one of SEQ ID NOs: 31-36.

In some embodiments, in the insulin analog, A₁ comprises (or is) the amino acid sequence of any one of SEQ ID NOs: 37-42.

In some embodiments, in the insulin analog, B₁ and A₁ respectively comprise (or are) the amino acid sequences set forth in any one of the following groups: SEQ ID NOs: 31 and 37; SEQ ID NOs: 32 and 38; SEQ ID NOs: 33 and 39; SEQ ID NOs: 34 and 40; SEQ ID NOs: 35 and 41; and SEQ ID NOs: 36 and 42.

In some embodiments, the linker represented by L₁ is a polypeptide capable of achieving a linking function, such as a flexible polypeptide. In some specific embodiments, the linker represented by L₁ comprises (or is) the following sequence: a sequence including but not limited to G₄S (SEQ ID NO: 53), GS, GAP, ASGS (SEQ ID NO: 54), and poly-guanine (poly G). In some specific embodiments, the linker represented by L₁ comprises (or is) (GₘSₚ)ₙ, wherein each m is independently selected from the group consisting of integers of 1-10 (e.g., 1, 2, 3, 4, 5, or 6), each n is independently selected from the group consisting of integers of 1-10 (e.g., 1, 2, 3, 4, 5, or 6), and each p is independently selected from the group consisting of 0-4. In some specific embodiments, there are at least five G in the GS or poly G linker.

In the present disclosure, the GS linker encompasses any type of linker that comprises the amino acids G and S.

In some specific embodiments, the linker represented by L₁ comprises (or is) GGSGGGG (SEQ ID NO: 45), GSGGGG (SEQ ID NO: 46), GGGGG (SEQ ID NO: 47), GGGGGGSGGGG (SEQ ID NO: 3), or GGGGGSGGGG (SEQ ID NO: 52).

In some embodiments, the fusion protein further comprises a C₁ domain capable of extending the half-life of the insulin analog.

In some embodiments, the fusion protein further comprises a C₁ domain selected from the group consisting of, for example, an Fc region of an immunoglobulin (e.g., IgG (e.g., IgG1, IgG2, IgG3 or IgG4 subtype), IgA1, IgA2, IgD, IgE, or IgM), an HSA protein or polypeptide, an HSA-binding domain (e.g., an anti-HSA antibody or an antigen-binding fragment thereof), an XTEN peptide, a glycine-rich homo-amino-acid polymer (HAP), a PAS polypeptide, an elastin-like polypeptide (ELP), a CTP peptide, and a gelatin-like protein (GLK) polymer.

In some specific embodiments, the XTEN peptide is an unstructured, hydrophilic long peptide comprising varying percentages of six amino acids: Ala, Glu, Gly, Ser, and Thr, such as the XTEN^{™} peptide (Amunix Operating Inc.). In some specific embodiments, the PAS polypeptide is a hydrophilic, uncharged polypeptide composed of Pro, Ala and Ser residues, with a length of at least about 100, 200, 300, 400, 500 or 600 amino acids. In some embodiments, one fusion protein comprises 1 or more (e.g., 2, 3, or 4) insulin analogs and one C₁.

In some specific embodiments, C₁ is selected from the group consisting of Fc regions of human IgG1, IgG2 and IgG4. In some specific embodiments, the Fc region comprises a mutation selected from the group consisting of: L234A/L235A on IgG1; V234A/G237A/P238S/H268A/V309L/A330S/P331S on IgG2; F234A/L235A on IgG4; S228P or S228P/F234A/L235A on IgG4; N297A on IgG1, IgG2, IgG3 or IgG4; V234A/G237A on IgG2; K214T/E233P/L234V/L235A/G236 deletion/A327G/P331A/ D365E/L358MV309L/A330S/P331S on IgG1; L234F/L235E/D265A on IgG1; L234A/L235A/G237A/P238S/H268A/A330S/P331S on IgG1; S228P/F234A/L235A/ G237A/P238S on IgG4; and S228P/F234A/L235A/G236 deletion/G237A/P238S on IgG4. A hybrid IgG2/4 Fc domain may also be used, such as an Fc with residues 117-260 from IgG2 and residues 261-447 from IgG4. In some specific embodiments, the Fc region of human IgG4 has the mutations S228P, F234A, L235A, and/or K447A. In some specific embodiments, the Fc region of human IgG1 has the mutations L234A/L235A or L234A/L235A/P329G. In some embodiments, the mutation positions of the Fc region described above are numbered according to the EU numbering scheme. In some specific embodiments, C₁ is selected from the group consisting of an amino acid sequence comprising (or as set forth in) any one of SEQ ID NOs: 17, 18, and 48-51, and a sequence having at least 80%, 90%, 95%, 96%, 97%, 98% or 99% identity thereto.

### > Human IgG1 Fc region 1

EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK (SEQ ID NO: 48);

### > human IgG1 Fc region 2

EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK (SEQ ID NO: 49);

### > human IgG4 Fc region 1

ESKYGPPCPPCPAPEFEGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK (SEQ ID NO: 50);

### > human IgG4 Fc region 2

ESKYGPPCPPCPAPEAAGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH

NHYTQKSLSLSLGK (SEQ ID NO: 51).

In some embodiments, the fusion protein further comprises L₂, the linker represented by L₂ is a polypeptide capable of achieving a linking function (e.g., a flexible polypeptide), and C₁ forms the fusion protein with B₁-L₁-A₁ via L₂. In some specific embodiments, the linker represented by L₂ comprises (or is) the following sequence: a sequence including but not limited to G₄S, GS, GAP, ASGS, poly-guanine (poly G), (GₘQ)ₙ, (GₘA)ₙ, (GₘQᵢ)ₙ, (GₘAᵢ)ₙ, or (PGPQ)ₛ (SEQ ID NO: 55), wherein each m is independently selected from the group consisting of integers of 1-10 (e.g., 1, 2, 3, 4, 5, or 6), each n is independently selected from the group consisting of integers of 1-10 (e.g., 1, 2, 3, 4, 5, or 6), each i is independently selected from the group consisting of 0-4 (e.g., 0, 1, 2, 3, or 4), and s is selected from the group consisting of integers of 1-10 (e.g., 1, 2, 3, 4, 5, or 6). In some specific embodiments, L₂ comprises (or is) the amino acid sequence set forth in any one of SEQ ID NOs: 10-16.

In some embodiments, in the fusion protein, C₁ is located at the N-terminus of the insulin analog. In some other embodiments, in the fusion protein, C₁ is located at the C-terminus of the insulin analog. In some specific embodiments, the fusion protein comprises, from the N-terminus to the C-terminus, B₁-L₁-A₁-L₂-C₁, C₁-L₂-B₁-L₁-A₁, A₁-L₁-B₁-L₂-C₁, or C₁-L₂-A₁-L₁-B₁.

In some embodiments, the insulin analog is an insulin receptor agonist having insulin receptor agonistic activity.

In some embodiments, the fusion protein is a monomer, a dimer, or a multimer. In some specific embodiments, the dimer is a homodimer, wherein the amino acid sequences of the two fusion proteins that make up the dimer are identical or substantially identical. In some other embodiments, the dimer is a heterodimer, wherein the amino acid sequences of the two fusion proteins that make up the dimer are different.

In some embodiments, the fusion protein comprises (or is) the amino acid sequence set forth in any one of SEQ ID NOs: 19-27, or a sequence having at least 80%, 90%, 95%, 96%, 97%, 98% or 99% identity thereto.

In some embodiments, the fusion protein of the present disclosure (e.g., the sequence of any one of SEQ ID NOs: 19-27, more specifically SEQ ID NO: 19, 20, 21 or 27) has a reduction in cell proliferation-promoting ability, thereby having reduced risk of inducing tumorigenesis and/or tumor development. The cell is, for example, a cell expressing IGF-1R on the surface. In some embodiments, the reduction means that the fusion protein, compared to natural human insulin, has an EC₅₀ value for promoting cell (e.g., MCF-7) proliferation that is increased by 1 to 10 times, 1 to 100 times, 1 to 1000 times, 1 to 10000 times, 100 to 500 times, or 200 to 400 times; for example, the EC₅₀ is increased by about 100 times, about 200 times, about 300 times, about 400 times, about 500 times, about 600 times, about 700 times, about 800 times, about 900 times, or about 1000 times. In some specific embodiments, the fusion protein 1 set forth in SEQ ID NO: 19 has a cell proliferation-promoting ability that is about 200 times lower than that of natural human insulin. The EC₅₀ for cell (e.g., MCF-7) proliferation can be assayed using the method in Example 5 of the present disclosure.

In some embodiments, the fusion protein of the present disclosure (e.g., the sequence of any one of SEQ ID NOs: 19-27, more specifically SEQ ID NO: 19 or 20) has a half-life of 1 day to 10 days, 2 days to 9 days, 2 days to 8 days, 3 days to 7 days, such as a half-life of about 1 day, about 2 days, about 2.5 days, about 3 days, about 3.5 days, about 4 days, about 4.5 days, about 5 days, about 5.5 days, about 6 days, about 6.5 days, about 7 days, about 7.5 days, about 8 days, about 8.5 days, about 9 days, about 9.5 days, or about 10 days. In some specific embodiments, the fusion protein 1 set forth in SEQ ID NO: 19 has a half-life of about 4.5 days. The half-life of the protein can be assayed using the method in Example 6 of the present disclosure.

### Polynucleotide

The present disclosure provides a polynucleotide encoding the fusion protein or the insulin analog of the present disclosure. The polynucleotide of the present disclosure may be an RNA, a DNA, or a cDNA. According to some embodiments of the present disclosure, the nucleic acid of the present disclosure is a substantially isolated nucleic acid.

"Substantially isolated" refers to being separated from its naturally occurring state or being in a purified state, and in this case means that the designated molecule is substantially free of other biomolecules or other materials (e.g., cell debris or culture medium). "Substantially isolated" is not intended to mean the complete absence of such materials or the absence of water, buffers, or salts, unless they are present in amounts that significantly interfere with therapeutic use.

The nucleic acid of the present disclosure may also be in the form of a vector, and it may be present in the vector and/or may be part of the vector. The vector is, e.g., a plasmid, cosmid, YAC, or viral vector. The vector may be particularly an expression vector, i.e., a vector that can provide expression of the fusion protein or the insulin analog *in vitro* and/or *in vivo* (i.e., in a suitable host cell, host organism and/or expression system). The expression vector generally comprises at least one of the nucleic acids of the present disclosure, which is operably linked to one or more suitable expression regulatory elements (e.g., promoters, enhancers, terminators, and the like). The selection of the elements and their sequences for expression in a particular host is within the knowledge of those skilled in the art. Regulatory elements and other elements useful or necessary for expression of the fusion protein or the insulin analog of the present disclosure include, for example, promoters, enhancers, terminators, integrons, selection labels, leader sequences, and reporter genes.

The nucleic acid of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

### Host Cell

The present disclosure provides a recombinant host cell expressing one or more of the fusion proteins or the insulin analogs of the present disclosure, or containing the polynucleotide or the vector of the present disclosure. In some embodiments, the host cell is a bacterial cell, a fungal cell, or a mammalian cell.

Bacterial cells include, for example, cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, for example, cells of species of *Trichoderma*, *Neurospora*, and *Aspergillus*; or cells of species of *Saccharomyces* (e.g., *Saccharomyces cerevisiae*), *Schizosaccharomyces* (e.g., *Schizosaccharomyces pombe*), *Pichia* (*Pichia pastoris* and *Pichia methanolica*), and *Hansenula.*

Mammalian cells include, for example, HEK293 cells, CHO cells, BHK cells, HeLa cells, COS cells, and the like.

However, amphibian cells, insect cells, plant cells, and any other cells used in the art for expressing heterologous proteins may also be used in the present disclosure.

The cells of the present disclosure are unable to develop into complete plants or individual animals.

### Production or Preparation Method

The present disclosure provides a method for preparing the fusion protein or the insulin analog of the present disclosure, which generally comprises the following steps:
- culturing the host cell of the present disclosure under conditions that allow expression of the fusion protein or the insulin analog of the present disclosure; and
- collecting the target fusion protein or insulin analog expressed by the host cell from the culture; and
- optionally, further purifying and/or modifying the target fusion protein or insulin analog of the present disclosure.

The fusion protein or the insulin analog of the present disclosure can be produced intracellularly (e.g., in the cytoplasm, in the periplasm, or in inclusion bodies) in the cell as described above, followed by isolation from the host cell and optionally further purification; or it may be produced extracellularly (e.g., in the medium in which the host cell is cultured), followed by isolation from the medium and optionally further purification.

Methods and reagents for recombinant production of polypeptides, e.g., specific suitable expression vectors, transformation or transfection methods, selection labels, methods for inducing protein expression, culture conditions, and the like, are known in the art. Similarly, protein isolation and purification techniques suitable for use in the method for producing the protein of the present disclosure are well known to those skilled in the art. As an example, cDNA sequences encoding the heavy and light chains can be cloned and recombined into an expression vector. Recombinant immunoglobulin expression vectors can be stably transfected into CHO cells. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium of a bioreactor to produce antibodies. The culture medium with the secreted antibody can be purified and collected by conventional techniques. The antibody can be filtered and concentrated by conventional methods. Soluble mixtures and multimers can also be removed by conventional methods, such as molecular sieves and ion exchange. The resulting products need to be immediately frozen, e.g., at -70 °C, or lyophilized. However, the fusion protein or the insulin analog of the present disclosure may also be obtained by other methods for producing proteins known in the art, such as chemical synthesis, including solid-phase or liquid-phase synthesis.

### Pharmaceutical Composition

The present disclosure provides a pharmaceutical composition comprising a prophylactically or therapeutically effective amount of any one selected from the group consisting of the following or a combination thereof: the fusion protein, the insulin analog and the polynucleotide encoding the same as described above, as well as one or more pharmaceutically acceptable carriers, diluents, buffers or excipients.

In some specific embodiments, the pharmaceutical composition may comprise from 0.01 to 99 wt% of the fusion protein or the insulin analog in a unit dose. In some other specific embodiments, the amount of the fusion protein or the insulin analog in a unit dose of the pharmaceutical composition is 0.1-2000 mg, and in some embodiments, 1-1000 mg.

### Kit (or Kit-of-Parts)

The present disclosure provides a kit or kit-of-parts, which comprises one or more containers each independently comprising any one selected from the group consisting of the following or a combination thereof: the fusion protein, the insulin analog, and the polynucleotide encoding the same of the present disclosure.

In some embodiments, the device comprises a syringe and a needle. In some specific embodiments, the device is a pre-filled syringe.

### Method for Preventing and Treating Disease and Pharmaceutical Use

The present disclosure provides use of the composition comprising the fusion protein or the insulin analog of the present disclosure, the polynucleotide, the pharmaceutical composition in the prevention, treatment, or alleviation of a disease or symptom, and a method related thereto.

In some embodiments, a method of administering an effective amount of the fusion protein or the insulin analog of the present disclosure to a subject in need thereof for preventing, treating or alleviating a disease or symptom is included.

In some embodiments, the disease or symptom is selected from the group consisting of non-insulin-dependent diabetes, insulin-dependent diabetes, and other metabolic diseases. The subject in need is untreated or is treated with oral drugs (such as sulfonylureas, metformin, thiazolidinediones like pioglitazone, and α-glucosidase inhibitors like acarbose) and/or non-insulin injectable agents (including incretin-based therapies such as DPP-4 inhibitors and GLP-1R agonists).

In some embodiments, the disease or symptom is selected from the group consisting of diabetes, obesity, dyslipidemia, and/or metabolic syndrome.

In some embodiments, the disease or symptom is a complication of diabetes, or diabetes-related heart disease, stroke, nephropathy, retinopathy, neuropathy or kidney disease.

In some embodiments, the disease or symptom is selected from the group consisting of hyperglycemia, type II diabetes, impaired glucose tolerance, type I diabetes, obesity, syndrome X, and dyslipidemia.

In some embodiments, a method of administering an effective amount of the fusion protein or the insulin analog of the present disclosure to a subject in need thereof for lowering blood glucose is provided.

The present disclosure further provides pharmaceutical use of the fusion protein or the insulin analog of the present disclosure in the preparation of a medicament for preventing, treating or alleviating the above disease or symptom.

### Definition

In order to facilitate the understanding of the present disclosure, some technical and scientific terms are specifically defined below. Unless otherwise specifically defined in the present disclosure, all other technical and scientific terms used in the present disclosure have the meanings generally understood by those of ordinary skill in the art to which the present disclosure pertains. The three-letter and single-letter codes for amino acids used in the present disclosure are as described in J. Biol. Chem, 243, p3558 (1968).

"Insulin" includes naturally occurring insulin (e.g., human insulin). Human insulin consists of two polypeptide chains, referred to as the A-chain and B-chain, which contain 21 and 30 amino acid residues, respectively, and are linked via two cysteine disulfide bonds. An exemplary A-chain is set forth in SEQ ID NO: 29 of the present disclosure, and an exemplary B-chain is set forth in SEQ ID NO: 30 of the present disclosure.

"Insulin analog" includes polypeptides that, through the removal and/or substitution (replacement) of one or more amino acid residues present in natural insulin and/or through the addition of one or more amino acid residues, have a molecular structure that can formally be derived from the structure of naturally occurring insulin (e.g., human insulin). The added and/or substituted amino acid residues may be codable amino acid residues, other naturally occurring amino acid residues, or purely synthetic amino acid residues.

"Insulin A-chain analog" refers to an analog that, relative to the A-chain of human insulin, has one or more amino acid substitutions, deletions and/or extensions (additions) on the A chain.

"Insulin B-chain analog" refers to an analog that, relative to the B chain of human insulin, has one or more amino acid substitutions, deletions and/or extensions (additions) on the B chain.

"Glucose-responsive insulin (GRI)" can regulate its release and action based on blood glucose levels, thereby achieving the purpose of reducing incidence of hypoglycemia and achieving more stable blood glucose control.

The term "insulin receptor agonist" refers to a protein that binds to and activates an insulin receptor, resulting in a decrease in blood glucose levels and/or inhibition of hepatic glucose output; it is characterized in that it can be tested and measured using known techniques (such as those shown in the studies described in the examples of the present disclosure). In some embodiments, the insulin receptor agonist is formed by directly or indirectly linking the insulin A-chain analog and the insulin B-chain analog (e.g., via a linker).

"Sequence" is generally understood to include both related amino acid sequences and nucleic acid or nucleotide sequences encoding the sequences, unless a further limited interpretation is required in the present disclosure.

"Homology" or "identity" refers to sequence similarity between two polynucleotide sequences or between two polypeptides. When positions in two compared sequences are occupied by identical bases or amino acid monomer subunits, e.g., if the position of each of two DNA molecules is occupied by adenine, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. Generally, when two sequences are aligned, a comparison is performed to obtain the maximum homology percentage.

The terms "nucleic acid molecule" and "polynucleotide" are used interchangeably, referring to a DNA molecule and an RNA molecule. The nucleic acid molecule may be single-stranded or double-stranded, and is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

"Vector" refers to a construct capable of delivering and expressing one or more target genes or sequences in a host cell. Examples of vectors include, but are not limited to, viral vectors, naked DNA or RNA expression vectors, plasmid, cosmid or phage vectors, DNA or RNA expression vectors bound to cationic condensing agents, and DNA or RNA expression vectors encapsulated in liposomes.

"Host cell" includes cells or cell cultures, which may be or have been the recipient of a vector. The host cell includes the progeny of a single host cell, and the progeny may not necessarily be identical (in morphology or genomic DNA complement) to the original parent cell due to natural, accidental or intentional mutations. The host cell includes cells transfected and/or transformed *in vivo* with the polynucleotide of the present disclosure. "Cell", "cell line", and "cell culture" are used interchangeably, and all such designations include their progeny (regardless of the number of passages); for example, mutant progeny that have the same function or biological activity as the parent cell selected from the initially transformed cells are included.

"Pharmaceutical composition" refers to a mixture containing one or more of the active ingredients or the physiologically/pharmaceutically acceptable salts or pro-drugs thereof described herein, and other chemical components, as well as other components, for example, physiologically/pharmaceutically acceptable carriers and excipients. The pharmaceutical composition is intended to promote administration to an organism and facilitate the absorption of the active ingredient so that it can exert its biological activity. "Pharmaceutically acceptable carrier, diluent or excipient" includes any material that, when combined with an active ingredient, allows the ingredient to retain its biological activity and does not have the intended therapeutic purpose. Examples include, but are not limited to, any standard pharmaceutical carrier, such as phosphate-buffered saline solutions, water, emulsions such as oil/water emulsions, and various types of wetting agents. In some embodiments, the diluent for aerosol or parenteral administration is phosphate-buffered saline (PBS) or normal (0.9%) saline. Compositions containing such carriers are prepared by well-known conventional methods (see, e.g., Remington's Pharmaceutical Sciences, 18th edition, A. Gennaro, eds., Mack Publishing Co., Easton, PA, 1990; and R Remington, The Science and Practice of Pharmacy, 20th edition, Mack Publishing, 2000).

"Giving", "administering" and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs or biological fluids, e.g., therapeutic, pharmacokinetic, diagnostic, research and experimental methods. The treatment of cells comprises contacting a reagent with cells and contacting the reagent with a fluid, where the fluid is in contact with the cells. "Giving", "administering", and "treating" also refer to treating, e.g., a cell, by a reagent, diagnosis, a binding composition, or by another cell *in vitro* and *ex vivo.* When applied to humans, veterinary medicine, or research subjects, they refer to therapeutic treatment, preventive or prophylactic measures, and research and diagnostic applications.

"Treat", "treating", or "treatment" refers to administering a therapeutic agent, such as a therapeutic agent comprising any one of the fusion proteins or the insulin analogs of the present disclosure, to a subject who has had, is suspected of having, or is predisposed to having one or more types of diabetes or hyperglycemia-related diseases or symptoms thereof on which the therapeutic agent is known to have a therapeutic effect. Generally, the therapeutic agent is administered in an amount effective to alleviate one or more disease symptoms in the subject or population being treated. This is achieved by preventing or delaying the onset of the symptoms or complications, alleviating the symptoms or complications, or eliminating the disease, condition or disorder to any clinically measurable degree. The amount of therapeutic agent effective to alleviate any particular disease symptom (also referred to as the "therapeutically effective amount") may vary depending on factors such as the disease state, age and body weight of the subject, and the ability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating the target disease symptoms in a certain subject, they shall alleviate the target disease symptoms in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test. The patient to be treated is a mammal, preferably a human.

"Prevent", "preventing", or "prevention" refers to reducing the risk or incidence of one or more conditions, symptoms, complications or disorders, or eliminating or slowing the progression of one or more conditions, symptoms, complications or disorders.

"Optional" or "optionally" means that the event or circumstance subsequently described may, but does not necessarily, occur, and this description includes instances where the event or circumstance occurs or does not occur.

"Subject" or "patient" means a mammal, particularly a primate, and especially a human. "About" or "approximately" means that a numerical value is within an acceptable error range for the specific value determined by those of ordinary skill in the art, and the numerical value depends in part on how the value is measured or determined (i.e., the limitations of the measurement system). For example, "about" may mean a standard deviation within 1 or more than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes embodiments of the given number. Unless otherwise stated, when a specific value is provided in the present application and claims, the meaning of "about" or "substantially comprise" should be assumed to be within an acceptable error range for that specific value.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

### BRIEF DESCRIPTION OF THE DRAWINGS

FIG. 1 shows the hypoglycemic effect after a single subcutaneous injection of fusion protein 1 in an STZ-induced type I diabetic rat model.
FIG. 2 shows the hypoglycemic effects after a single subcutaneous injection of LY3209590, fusion protein 2 and fusion protein 9 in an STZ-induced type I diabetic rat model.

### DETAILED DESCRIPTION

The present disclosure is further described with reference to the following examples, but these examples are not intended to limit the scope of the present disclosure. Experimental procedures without specific conditions indicated in the examples or test examples of the present disclosure are generally conducted under conventional conditions, or under conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated are commercially available conventional reagents.

### Example 1. Design of Human Insulin Analogs and Their Fusion Proteins with Fc

This example provides a human insulin analog with the following general formula from the N-terminus to the C-terminus: B₁-L₁-A₁, wherein

B₁ is a human insulin B-chain analog with the following sequence:
FVX₁QHLCGX₂HLVEALX₃X₄VCGERGFX₅Y (SEQ ID NO: 1), wherein X₁ is selected from the group consisting of the amino acid residues of Asn, Gly and Lys, X₂ is selected from the group consisting of the amino acid residues of Ser and Glu, X₃ is selected from the group consisting of the amino acid residues of Tyr, His and Glu, X₄ is selected from the group consisting of the amino acid residues of His, Arg, Leu and Glu, and X₅ is selected from the group consisting of the amino acid residues of Phe and His;
A₁ is an insulin A-chain analog with the following sequence: GIVEQCCZ₁SICSLZ₂QLENYCZ₃ (SEQ ID NO: 2), wherein Z₁ is selected from the group consisting of the amino acid residues of Thr, His and Glu, Z₂ is selected from the group consisting of the amino acid residues of Tyr, Asp, Ser and Glu, and Z₃ is selected from the group consisting of the amino acid residues of Gly and Asn;
L₁ is GGGGGGSGGGG (SEQ ID NO: 3) or GGGGGSGGGG (SEQ ID NO: 52).

The sequences of exemplary human insulin analogs are as follows:
> human insulin analog 1 
FVNQHLCGEHLVEALYLVCGERGFHYGGGGGGSGGGGGIVEQCCESICSLEQLE
NYCG (SEQ ID NO: 4);
> human insulin analog 2 
FVNQHLCGSHLVEALYLVCGERGFHYGGGGGGSGGGGGIVEQCCHSICSLEQLE
NYCG (SEQ ID NO: 5);
> human insulin analog 3 
FVNQHLCGSHLVEALHLVCGERGFHYGGGGGGSGGGGGIVEQCCTSICSLEQLE
NYCN (SEQ ID NO: 6);
> human insulin analog 4 
FVKQHLCGEHLVEALYLVCGERGFHYGGGGGGSGGGGGIVEQCCESICSLEQLE
NYCG (SEQ ID NO: 7);
> human insulin analog 5 
FVNQHLCGEHLVEALYLVCGERGFHYGGGGSGGGGGIVEQCCESICSLEQLEN
YCG (SEQ ID NO: 8);
> human insulin analog 6

FVNQHLCGEHLVEALYLVCGERGFHYGGGGGGSGGGGGIVEQCCESICSLEQLE
NYCN (SEQ ID NO: 9).

Further, fusion proteins of the above human insulin analogs with Fc are provided, with the fusion of the two being achieved via linkers as shown in Table 1.

**Table 1. Linker sequences for fusion of human insulin analogs with Fc**

| Amino acid sequence | Sequence No. |
|---|---|
| GGGGQGGGGQGGGGQGGGGG | SEQ ID NO:10 |
| HGGGQGGGGQGGGGQGGGGG | SEQ ID NO:11 |
| GGGGQGGGGQGGGGQGGGGQGGGGQ | SEQ ID NO:12 |
| PGPQPGPQPGPQPGPQPGPQPGPQPGPQPGPQ | SEQ ID NO:13 |
| GGGGQGGGGQGGGGQGGGGGQGGGG | SEQ ID NO:14 |
| GGGGAGGGGAGGGGAGGGGG | SEQ ID NO:15 |
| GGGGQGGGGQGGGGQGGGGQGGPCPPCPAPEAAG | SEQ ID NO:16 |

The Fc is the Fc of human IgG2, with the following amino acid sequences:
> IgG2 Fc1 
ECPPCPAPPVAGPSVFLFPPKPKDTLMISRTPEVTCVVVDVSHEDPEVQFNWYVD
GVEVHNAKTKPREEQFNSTFRVVSVLTVVHQDWLNGKEYKCKVSNKGLPAPIE
KTISKTKGQPREPQVYTLPPSREEMTKNQVSLTCLVKGFYPSDIAVEWESNGQPE
NNYKTTPPMLDSDGSFFLYSKLTVDKSRWQQGNVFSCSVMHEALHNHYTQKSL
SLSPG (SEQ ID NO: 17);
> IgG2 Fc2

GPSVFLFPPKPKDTLMISRTPEVTCVVVDVSHEDPEVQFNWYVDGVEVHNAKT
KPREEQFNSTFRVVSVLTVVHQDWLNGKEYKCKVSNKGLPAPIEKTISKTKGQP
REPQVYTLPPSREEMTKNQVSLTCLVKGFYPSDIAVEWESNGQPENNYKTTPPM
LDSDGSFFLYSKLTVDKSRWQQGNVFSCSVMHEALHNHYTQKSLSLSPG
(SEQ ID NO: 18).

The amino acid sequences of exemplary fusion proteins are as follows:
> fusion protein 1 (insulin analog 1 + L2 + IgG2 Fc1) 
FVNQHLCGEHLVEALYLVCGERGFHYGGGGGSGGGGGIVEQCCESICSLEQLE
NYCGGGGGQGGGGQGGGGQGGGGGECPPCPAPPVAGPSVFLFPPKPKDTLMIS
RTPEVTCVVVDVSHEDPEVQFNWYVDGVEVHNAKTKPREEQFNSTFRVVSVLT
VVHQDWLNGKEYKCKVSNKGLPAPIEKTISKTKGQPREPQVYTLPPSREEMTK
NQVSLTCLVKGFYPSDIAVEWESNGQPENNYKTTPPMLDSDGSFFLYSKLTVDK
 SRWQQGNVFSCSVMHEALHNHYTQKSLSLSPG (SEQ ID NO: 19);
> fusion protein 2 (insulin analog 2 + L2 + IgG2 Fc1) 
FVNQHLCGSHLVEALYLVCGERGFHYGGGGGGSGGGGGIVEQCCHSICSLEQLE
NYCGGGGGQGGGGQGGGGQGGGGGECPPCPAPPVAGPSVFLFPPKPKDTLMIS
RTPEVTCVVVDVSHEDPEVQFNWYVDGVEVHNAKTKPREEQFNSTFRVVSVLT
VVHQDWLNGKEYKCKVSNKGLPAPIEKTISKTKGQPREPQVYTLPPSREEMTK
NQVSLTCLVKGFYPSDIAVEWESNGQPENNYKTTPPMLDSDGSFFLYSKLTVDK
SRWQQGNVFSCSVMHEALHNHYTQKSLSLSPG (SEQ ID NO: 20);
> fusion protein 3 (insulin analog 3 + L2 + IgG2 Fc1) 
FVNQHLCGSHLVEALHLVCGERGFHYGGGGGGSGGGGGIVEQCCTSICSLEQLE
NYCNGGGGQGGGGQGGGGQGGGGGECPPCPAPPVAGPSVFLFPPKPKDTLMIS
RTPEVTCVVVDVSHEDPEVQFNWYVDGVEVHNAKTKPREEQFNSTFRVVSVLT
VVHQDWLNGKEYKCKVSNKGLPAPIEKTISKTKGQPREPQVYTLPPSREEMTK
NQVSLTCLVKGFYPSDIAVEWESNGQPENNYKTTPPMLDSDGSFFLYSKLTVDK
SRWQQGNVFSCSVMHEALHNHYTQKSLSLSPG (SEQ ID NO: 21);
> fusion protein 4 (insulin analog 4 + L2 + IgG2 Fc1) 
FVKQHLCGEHLVEALYLVCGERGFHYGGGGGSGGGGGIVEQCCESICSLEQLE
NYCGGGGGQGGGGQGGGGQGGGGGECPPCPAPPVAGPSVFLFPPKPKDTLMIS
RTPEVTCVVVDVSHEDPEVQFNWYVDGVEVHNAKTKPREEQFNSTFRVVSVLT
VVHQDWLNGKEYKCKVSNKGLPAPIEKTISKTKGQPREPQVYTLPPSREEMTK
NQVSLTCLVKGFYPSDIAVEWESNGQPENNYKTTPPMLDSDGSFFLYSKLTVDK
SRWQQGNVFSCSVMHEALHNHYTQKSLSLSPG (SEQ ID NO: 22);
> fusion protein 5 (insulin analog 1 + L2 + IgG2 Fc1) 
FVNQHLCGEHLVEALYLVCGERGFHYGGGGGGSGGGGGIVEQCCESICSLEQLE
NYCGGGGGQGGGGQGGGGQGGGGQGGGGQECPPCPAPPVAGPSVFLFPPKPK
DTLMISRTPEVTCVVVDVSHEDPEVQFNWYVDGVEVHNAKTKPREEQFNSTFR
VVSVLTVVHQDWLNGKEYKCKVSNKGLPAPIEKTISKTKGQPREPQVYTLPPSR
EEMTKNQVSLTCLVKGFYPSDIAVEWESNGQPENNYKTTPPMLDSDGSFFLYSK
LTVDKSRWQQGNVFSCSVMHEALHNHYTQKSLSLSPG (SEQ ID NO: 23);
> fusion protein 6 (insulin analog 5 + L2 + IgG2 Fc1) 
FVNQHLCGEHLVEALYLVCGERGFHYGGGGGSGGGGGIVEQCCESICSLEQLEN
YCGGGGGQGGGGQGGGGQGGGGGECPPCPAPPVAGPSVFLFPPKPKDTLMISR
TPEVTCVVVDVSHEDPEVQFNWYVDGVEVHNAKTKPREEQFNSTFRVVSVLTV
VHQDWLNGKEYKCKVSNKGLPAPIEKTISKTKGQPREPQVYTLPPSREEMTKN
QVSLTCLVKGFYPSDIAVEWESNGQPENNYKTTPPMLDSDGSFFLYSKLTVDKSR
WQQGNVFSCSVMHEALHNHYTQKSLSLSPG (SEQ ID NO: 24);
> fusion protein 7 (insulin analog 1 + L2 + IgG2 Fc1) 
FVNQHLCGEHLVEALYLVCGERGFHYGGGGGSGGGGGIVEQCCESICSLEQLE
NYCGPGPQPGPQPGPQPGPQPGPQPGPQPGPQPGPQECPPCPAPPVAGPSVFLFPP
KPKDTLMISRTPEVTCVVVDVSHEDPEVQFNWYVDGVEVHNAKTKPREEQFNS
TFRVVSVLTVVHQDWLNGKEYKCKVSNKGLPAPIEKTISKTKGQPREPQVYTLP
 
PSREEMTKNQVSLTCLVKGFYPSDIAVEWESNGQPENNYKTTPPMLDSDGSFFL
YSKLTVDKSRWQQGNVFSCSVMHEALHNHYTQKSLSLSPG (SEQ ID NO: 25);
> fusion protein 8 (insulin analog 1 + L2 + IgG2 Fc1) 
FVNQHLCGEHLVEALYLVCGERGFHYGGGGGGSGGGGGIVEQCCESICSLEQLE
NYCGGGGGAGGGGAGGGGAGGGGGECPPCPAPPVAGPSVFLFPPKPKDTLMIS
RTPEVTCVVVDVSHEDPEVQFNWYVDGVEVHNAKTKPREEQFNSTFRVVSVLT
VVHQDWLNGKEYKCKVSNKGLPAPIEKTISKTKGQPREPQVYTLPPSREEMTK
NQVSLTCLVKGFYPSDIAVEWESNGQPENNYKTTPPMLDSDGSFFLYSKLTVDK
SRWQQGNVFSCSVMHEALHNHYTQKSLSLSPG (SEQ ID NO: 26);
> fusion protein 9 (insulin analog 6 + L2 + IgG2 Fc1)

FVNQHLCGEHLVEALYLVCGERGFHYGGGGGSGGGGGIVEQCCESICSLEQLE
NYCNHGGGQGGGGQGGGGQGGGGGECPPCPAPPVAGPSVFLFPPKPKDTLMIS
RTPEVTCVVVDVSHEDPEVQFNWYVDGVEVHNAKTKPREEQFNSTFRVVSVLT
VVHQDWLNGKEYKCKVSNKGLPAPIEKTISKTKGQPREPQVYTLPPSREEMTK
NQVSLTCLVKGFYPSDIAVEWESNGQPENNYKTTPPMLDSDGSFFLYSKLTVDK
SRWQQGNVFSCSVMHEALHNHYTQKSLSLSPG (SEQ ID NO: 27).

In addition, LY3209590, developed by Eli Lilly and Company, USA, is an ultra-long-acting insulin analog administered once a week, which is currently in Phase III clinical trials, and has the following amino acid sequence: 
FVNQHLCGSHLVEALELVCGERGFHYGGGGGGSGGGGIVEQCCTSTCSLDQL
ENYCGGGGGQGGGGQGGGGQGGGGGECPPCPAPPVAGPSVFLFPPKPKDTLMI
SRTPEVTCVVVDVSHEDPEVQFNWYVDGVEVHNAKTKPREEQFNSTFRVVSVL
TVVHQDWLNGKEYKCKVSNKGLPAPIEKTISKTKGQPREPQVYTLPPSREEMT
KNQVSLTCLVKGFYPSDIAVEWESNGQPENNYKTTPPMLDSDGSFFLYSKLTV
DKSRWQQGNVFSCSVMHEALHNHYTQKSLSLSPG (SEQ ID NO: 28);
 linker sequences are all underlined.

The wild-type human insulin sequences are as follows:
> A-chain
   GIVEQCCTSICSLYQLENYCN (SEQ ID NO: 29);
> B-chain
   FVNQHLCGSHLVEALYLVCGERGFFYTPKT (SEQ ID NO: 30).

### Example 2. Preparation, Characterization, and Identification of Human Insulin Analog-Fc Fusion Proteins

### 1. Preparation

### 1) Expression in HEK-293 cells

A correctly cloned plasmid was extracted using an endotoxin removal midiprep kit (Promega, A2495), and after correct sequencing, preparations were made for cell transfection to produce the protein. HEK293 suspension cells were routinely passaged using Freestyle 293 medium (Invitrogen, cat No. 12338026). One day before transfection, the cells were passaged to a density of 6 × 10⁵ cells/mL. On the day of transfection, cell activity and density were measured to ensure that the cell activity was greater than 90%, and the cell density was adjusted to 1.1 × 10⁶ cells/mL. Plasmid DNA was prepared in an amount of 1 µg of DNA per mL of cells. The DNA used and the corresponding 293-fectin (Invitrogen, cat No. 12347019) were each diluted using opti-MEM (Invitrogen, cat No. 51985-034), shaken gently for mixing well, and left to stand at room temperature for 5 min. The diluted DNA and liposome were then mixed by gentle shaking, left to stand at room temperature for 20 min, and then slowly added to the HEK293 cells to be transfected. The cell culture flask was gently swirled. The cells were then placed in a shaker at 37 °C for suspension shaking culture (5% CO₂, 135 rpm, 75% humidity). 5 days after transfection, the supernatant of cell culture medium was collected and centrifuged at 4000 rpm for 20 min to collect a supernatant, which was then filtered using a 0.45 µm filter.

### 2) Expression in ExpiCHO cells

ExpiCHO suspension cells were routinely passaged using the ExpiCHO expression medium (Invitrogen, Cat No. A2910002) and transfected with the ExpiCHO expression system (Cat. No. A29133) at a ratio of 1 µg of DNA per mL of transfected cells. Using the "High protocol" from the manual, the cells were placed in a shaker at 32 °C for suspension shaking culture (5% CO₂, 110 rpm, 75% humidity) after transfection. On day 10-12 after transfection, the supernatant of cell culture medium was collected and centrifuged at 4000 rpm for 20 min to collect a supernatant, which was then filtered using a 0.45 µm filter.

### 3) Detection of expression level

Each human insulin analog-Fc fusion protein was tested for its OD280 after purification using a UV absorption method, and based on this, the expression levels of each fusion protein in different expression systems were calculated. The results show that fusion proteins 1 to 9 could all be expressed in the HEK293 expression system, with some of the fusion proteins exhibiting excellent yields. For example, fusion protein 1 exhibited a yield of 119 mg/L, fusion protein 4 exhibited a yield of 116 mg/L, fusion protein 6 exhibited a yield of 132 mg/L, fusion protein 7 exhibited a yield of 110 mg/L, and fusion protein 8 exhibited a yield of 119 mg/L. In the ExpiCHO transient expression system, the expression level of fusion protein 1 exceeded 400 mg/L.

### 4) Purification

### Protein-A affinity chromatography:

The cell culture supernatant expressing the Fc fusion protein was centrifuged at high speed to collect a supernatant, which was then filtered using a 0.22 µm filter membrane. A Protein-A affinity column (GE, Cat: 17-5474-99) was regenerated with 5 column volumes of 0.1 M aqueous NaOH (Sigma: Cat: 71687-500g), and then washed and equilibrated with 5 column volumes of 1× PBS buffer (pH 7.4) (Sangon Biotech (Shanghai) Co., Ltd.: Cat. E607016-0500). The supernatant was loaded at a low flow rate to facilitate binding, with the flow rate controlled to ensure a retention time of at least about 1 min. After the binding was completed, the chromatography column was rinsed with 5-10 column volumes of 1× PBS buffer (pH 7.4) until the UV absorbance fell back to baseline. Then, the sample was eluted using a 0.1 M glycine buffer (pH 3.0) (Sigma: Cat. 410225-250G). The elution peaks were collected based on UV detection, and the eluted product was rapidly adjusted to pH 7-8 with 1 M Tris-HCl (pH 7.5) (Vetec, Cat. V900483) and temporarily stored. For the eluted product, solution exchange could be performed by methods well known to those skilled in the art, such as ultrafiltration concentration using an ultrafiltration tube to exchange the solution to a desired buffer system, or replacement with a desired buffer system by size exclusion (e.g., G-25 desalination). The final storage system for the human insulin analog-Fc fusion protein was 1× PBS buffer at pH 7.4.

### Anion chromatography:

Firstly, the sample was diluted with an equilibration buffer of 20 mM Tris pH 8.0 (Vetec, Cat. V900483) to allow its conductivity to be lower than 3 ms/cm, and the Q HP (GE, Cat: 17-1014-01) anion chromatography column was regenerated with 5 column volumes of a 0.5 M aqueous NaOH solution (Sigma Cat: 71687-500g), and then washed and equilibrated with 15 column volumes of 20 mM Tris buffer pH 8.0 (Vetec, Cat. V900483). The supernatant was loaded at a low flow rate to facilitate binding, with the flow rate controlled to ensure a retention time of at least 2 min. After the binding was completed, the chromatography column was rinsed with 10 column volumes of 20 mM Tris buffer pH 8.0 (Vetec, Cat. V900483) until the UV absorbance fell back to baseline. 0-100% gradient elution was performed with an elution buffer of 20 mM Tris pH 8.0 (Vetec, Cat. V900483) and 0.5 M NaCl (Vetec, Cat. V900058); the elution peaks were collected based on SEC-HPLC (Column: TSKgel G3000SW_{XL}, 5 µm, Cat. 008541) purity determination. For the eluted product, solution exchange could be performed by methods well known to those skilled in the art, such as ultrafiltration concentration using an ultrafiltration tube to exchange the solution to a desired buffer system, or replacement with a desired buffer system by size exclusion (e.g., G-25 desalination). The final storage buffer system for the human insulin analog-Fc fusion protein was 1× PBS buffer at pH 7.4.

### 2. Characterization and identification

Size exclusion chromatography (SEC-HPLC): The Agilent 1260 infinity high-performance liquid chromatography system was adopted, with Tosoh (TSGel-G3000SWXL, chromatographic column dimensions: 7.8 mm × 300 mm) used as a chromatographic column. Before injection, the chromatographic column and system were equilibrated with 2× PBS buffer containing 5% isopropanol. Then, 15 µg of the sample was injected and eluted using the same buffer. The method duration was 20 min, with a flow rate of 1 mL/min.

Ultra-high-performance liquid chromatography-mass spectrometry: Characterization was performed using the Agilent 1290-6530 ultra-high-performance liquid chromatography-mass spectrometry system, with Sepax-C4 (particle size: 3 µm, chromatographic column dimensions: 2.1 mm × 50 mm) used as a chromatographic column, wherein mobile phase A was an aqueous solution containing 0.1% formic acid, mobile phase B was acetonitrile containing 0.1% formic acid, the flow rate was 1 mL/min, the temperature of the chromatographic column was maintained at 60 °C, and the chromatographic gradient used is shown in Table 2. Mass spectrometer-related detection parameter settings: 1 GHz, capillary voltage: 5000 V, desolvation gas temperature: 350 °C, gas flow rate: 12 L/min, Fragmentor voltage: 380 V, MS acquisition range: 100 m/z-4000 m/z.

**Table 2. Chromatographic gradient**

| | | | | | | | | | | | |
|---|---|---|---|---|---|---|---|---|---|---|---|
| RT (min) | 0 | 2 | 7 | 7.1 | 8 | 8.5 | 9 | 9.5 | 10 | 10.5 | 12 |
| B% | 10 | 10 | 50 | 90 | 90 | 10 | 90 | 10 | 90 | 10 | 10 |

The data in Table 3 show fusion proteins 1 to 9 all had good purity and expected molecular weights after purification.

**Table 3. SEC-HPLC purity and molecular weight**

| Fusion Protein | SEC-HPLC purity | Measured molecular weight |
|---|---|---|
| LY3209590 | 100.0% | 66980.00 Da |
| Fusion protein 1 | 100.0% | 67241.36 Da |
| Fusion protein 2 | 100.0% | 67172.00 Da |
| Fusion protein 3 | 98.7% | 67164.00 Da |
| Fusion protein 4 | 99.1% | 67269.00 Da |
| Fusion protein 5 | 98.8% | 68095.00 Da |
| Fusion protein 6 | 99.3% | 67126.00 Da |
| Fusion protein 7 | 99.6% | 70602.00 Da |
| Fusion protein 8 | 99.5% | 66899.00 Da |
| Fusion protein 9 | 99.5% | 67515.00 Da |

In the following examples, the biological activity of the fusion proteins of the present disclosure was assayed.

### Example 3. Assay on Autophosphorylation of Type B Human Insulin Receptor (hIR-B)

The activities of different human insulin analog-Fc fusion proteins in promoting the autophosphorylation of the human insulin receptor B subtype were examined and compared in CHO-K1 cells overexpressing the human insulin receptor B subtype. The activity of the fusion proteins in promoting the autophosphorylation of the human insulin receptor B subtype was assessed by calculating the EC₅₀ values obtained in this test system, with lower EC₅₀ values indicating higher *in-vitro* activity.

Experimental method: 18 h before the start of the experiment, CHO-K1-hIRB cells (HD Biosciences Shanghai, CHO-K1-hIRB-M-20170706) were plated on a 96-well plate at 10⁴ cells/well, and 100 µL of growth medium (F-12K medium, 1x penicillin-streptomycin containing 10% FBS and 4 µg/mL blasticidin) was added. The cells were cultured at 37 °C with 5% CO₂ overnight. On the morning of the day of the experiment, each human insulin analog-Fc fusion protein was diluted as required in 50 µL of starvation medium (F-12K medium, 1× penicillin-streptomycin containing 0.1% BSA and 4 µg/mL blasticidin). After the old medium was discarded, the cells were incubated with the starvation medium containing the human insulin analog-Fc fusion protein at 37 °C with 5% CO₂ for 20 min. Subsequently, the medium was discarded, and the cells were lysed using a cell lysis buffer from a kit (IR-beta phospho-Y1150/1151 kit, Cisbio, Lot No. 63ADK016PEG) and incubated at 37 °C for 30 min. Then, 16 µL of the lysate was pipetted into a 96-well plate, and 4 µL of the prepared antibody mixture (containing Phospho InsuLin Receptor beta Cryptate antibody and Phospho InsuLin Receptor beta d2 antibody) was added. The resulting mixture was incubated at room temperature for 4 h.

Data processing method: Signal reading was performed using a Tecan microplate reader with an excitation wavelength of 320 nm and emission wavelengths of 620 nm and 665 nm. The signal ratios (665 nm/620 nm × 10,000) were calculated and fitted non-linearly to the sample concentrations using a four-parameter equation in GraphPad Prism 6 to obtain EC₅₀ values, as shown in Table 4. In this example and subsequent examples, the human insulin used as a control was purchased from Sigma-Aldrich.

**Table 4. Autophosphorylation activity of type B insulin receptor promoted by human insulin analog-Fc fusion proteins**

| Protein | Activity of human insulin receptor subtype B (EC₅₀) |
|---|---|
| Human insulin | 0.43 nM |
| LY3209590 | 115.50 nM |
| Fusion protein 1 | 421.60 nM |
| Fusion protein 2 | 2.68 nM |
| Fusion protein 3 | 4.57 nM |
| Fusion protein 4 | 288.00 nM |
| Fusion protein 5 | 181.80 nM |
| Fusion protein 6 | 328.50 nM |
| Fusion protein 7 | 275.00 nM |
| Fusion protein 8 | 326.30 nM |
| Fusion protein 9 | 119.70 nM |

The results in Table 4 show that the fusion proteins of the present disclosure all had certain activity in activating the autophosphorylation of the insulin receptor B subtype; the EC₅₀ values indicate that the activity of all fusion proteins was significantly weaker than that of natural human insulin, and the activity of some of the fusion proteins in activating the autophosphorylation of insulin receptor B subtype was comparable to or slightly stronger than that of LY3209590. This indicates that the disclosed fusion proteins are all human insulin receptor agonists, but compared to natural human insulin, the fusion proteins have reduced activity, providing a safer administration window in clinical practice and reducing related side effects such as hypoglycemia in patients.

### Example 4. Assay on AKT Phosphorylation Activity in C2C12 Mouse Myoblasts

Insulin analogs, upon binding to the insulin receptor, activate a series of downstream signaling pathways, among which the stimulation of AKT phosphorylation is one of the major signaling pathways for insulin and analogs thereof to exert their hypoglycemic effects. Therefore, the activities of different human insulin analog-Fc fusion proteins in stimulating AKT phosphorylation were examined and compared in C2C12 mouse myoblasts. The activity of the fusion proteins in stimulating AKT phosphorylation in mouse myoblasts was assessed by calculating the EC₅₀ values for the fusion proteins in this test system, with lower EC₅₀ values indicating higher activity.

Experimental method: 18 h before the start of the experiment, C2C12 cells (Procell, CL-0044) were plated on a 96-well plate at 10⁴ cells/well, and 100 µL of growth medium (DMEM medium, 1× penicillin-streptomycin containing 10% FBS) was added. The cells were cultured at 37 °C with 5% CO₂ overnight. On the morning of the day of the experiment, each human insulin analog-Fc fusion protein was diluted as required in 50 µL of starvation medium (DMEM medium, 1× penicillin-streptomycin without FBS). After the old medium was discarded, the cells were incubated with the starvation medium containing the human insulin analog-Fc fusion protein in an incubator at 37 °C with 5% CO₂ for 20 min. Subsequently, the medium was discarded, and the cells were lysed using a cell lysis buffer from a kit (AKT phospho-S473 kit, Cisbio, 64AKSPEG) and incubated at 37 °C for 30 min. Then, 16 µL of the lysate was pipetted into a 96-well plate, and 4 µL of the prepared antibody mixture (containing Phospho-AKT Eu Cryptate antibody and Phospho-AKT d2 antibody) was added. The resulting mixture was incubated at room temperature for 4 h. Using the data processing method in Example 3, EC₅₀ values were obtained, as shown in Table 5.

**Table 5. AKT phosphorylation activity stimulated by human insulin analog-Fc fusion proteins in C2C12 mouse myoblasts**

| Protein | AKT phosphorylation activity (EC₅₀) |
|---|---|
| Human insulin | 5.44 nM |
| LY3209590 | 193.80 nM |
| Fusion protein 1 | 489.90 nM |
| Fusion protein 2 | 90.49 nM |
| Fusion protein 3 | 28.56 nM |
| Fusion protein 4 | 565.10 nM |
| Fusion protein 5 | 320.00 nM |
| Fusion protein 6 | 455.70 nM |
| Fusion protein 7 | 280.00 nM |
| Fusion protein 8 | 543.80 nM |
| Fusion protein 9 | 132.90 nM |

The results show that the fusion proteins of the present disclosure all had certain activity in activating the AKT phosphorylation of the insulin receptor; the EC₅₀ values indicate that the activity of all fusion proteins was significantly weaker than that of natural human insulin, and the activity of some of the fusion proteins was comparable to that of LY3209590. This indicates that the fusion proteins all have significant activity in stimulating AKT phosphorylation in mouse myoblasts, but the activity is weaker than that of natural human insulin.

### Example 5. Assay on Promotion of Human MCF-7 (Human Breast Cancer Cell) Proliferation

The ability of some human insulin analog-Fc fusion proteins of the present disclosure to activate downstream signaling pathways to promote cell proliferation was examined in human MCF-7 breast cancer cells. For insulin and insulin analogs, many studies have shown that in addition to their induced hypoglycemic effects and other metabolic activities, insulin and insulin analogs also have the activity in activating other downstream signaling pathways to promote cell proliferation. The activity in promoting cell proliferation is closely related to the phenomenon of insulin and analogs thereof inducing tumorigenesis. The late-stage development of the rapid-acting insulin analog B10 Asp insulin, developed by Novo Nordisk, was terminated due to the significant tumorigenesis caused by this compound observed in a rat model. Therefore, the purpose of this experiment is to assess the ability of the fusion proteins of the present disclosure to induce cell proliferation using MCF-7 human breast cancer cells. A greatly reduced cell proliferation-promoting ability indicates a lower risk of inducing tumorigenesis in the later stages. Studies have shown that the cell proliferation-promoting ability of insulin or analogs thereof is related to the expression of IGF-1R on the surface of certain cells, and that insulin or analogs thereof react with IGF-1R to promote cell proliferation, thereby inducing the possibility of tumorigenesis and tumor development. Experimental method: Before the start of the experiment, MCF-7 cells (Procell, CL-147) were resuspended in a DMEM medium containing 0.5% FBS and counted, and 100 µL of the cell suspension was added to each well of a 96-well clear-bottom black cell culture plate at a density of 5 × 10³ cells/well. The cells were cultured at 37 °C with 5% CO₂ overnight. On the morning of the day of the experiment, each human insulin analog-Fc fusion protein was diluted as required in 50 µL of starvation medium (DMEM medium, containing 0.5% FBS). After the old medium was discarded, the cells were incubated with the starvation medium containing the human insulin analog-Fc fusion protein at 37 °C with 5% CO₂ for 3 days. After the incubation was completed, a CellTiter-Glo assay solution (50 µL) was added to the 96-well plate, and the mixture was shaken at room temperature for 2 min for mixing well and then left to stand in the dark for 10 min. The fluorescence values were then read using a Tecan microplate reader, and the EC₅₀ values were calculated, as shown in Table 6.

**Table 6. Activity of human insulin analog-Fc fusion proteins in promoting MCF-7 proliferation**

| Protein | Activity in promoting MCF-7 proliferation (EC₅₀) |
|---|---|
| Human insulin | 1.32 nM |
| LY3209590 | 31.24 nM |
| Fusion protein 1 | 280.30 nM |

The results show that, in multiple parallel experiments (one of which is shown in Table 6), fusion protein 1 consistently exhibited weaker activity in stimulating MCF-7 cell proliferation compared to natural human insulin. Unexpectedly, compared to LY3209590, fusion protein 1 had significantly reduced activity in stimulating the proliferation of MCF-7 human breast cancer cells, suggesting that this fusion protein had a potentially lower risk of inducing tumorigenesis. Meanwhile, the other fusion proteins 2 to 9 of the present disclosure also showed lower activity in promoting MCF-7 cell proliferation compared to human insulin. For example, the EC₅₀ for the activity of fusion protein 2 in promoting MCF-7 proliferation was about twice that of LY3209590.

### Example 6. Assay on Pharmacokinetic Properties of Human Insulin Analog-Fc Fusion Proteins

In this example, SD rats were used as an animal model to study the pharmacokinetic properties of fusion protein 1 after a single subcutaneous or intravenous injection in normal male SD rats.

Experimental method: Male SD rats (purchased from Vital River) weighing 170-200 g and aged 5-7 weeks were selected. Fusion protein 1 was prepared using 1× PBS buffer and then administered to the rats via tail vein injection or dorsal subcutaneous injection at a dose of 30 nmol/kg. The rats were not fasted after administration. Blood samples were collected from the orbit at 15 min, 1 h, 2 h and 4 h, and on day 1, day 3, day 5, day 8, day 11, day 14, day 21 and day 25 after administration, then anticoagulated with EDTA, and centrifuged to obtain plasma, which was used for the analysis of *in-vivo* pharmacokinetic properties. The analysis of pharmacokinetic properties was performed using an enzyme-linked immunosorbent assay. A multi-well plate was coated with a capture antibody against human IgG-Fc (I2136, Sigma). An insulin polyclonal antibody (337E2A20, Invitrogen) was added, which specifically bound to the test molecule.

Then, the concentration of the test molecule in the plasma was quantified using a detection antibody, anti-rabbit IgG peroxidase (A0545, Sigma). The standard curve and samples used in the assay were prepared using 1% rat plasma (EDTA). The results are shown in Tables 7 and 8.

**Table 7. Time-dependent changes in plasma concentration of fusion protein 1 in SD rats**

| Time | Plasma concentration (nM) | |
|---|---|---|
| | Intravenous injection | Subcutaneous injection |
| 15min | 1455±73.0 | 2.58±0.80 |
| 1h | 1123±11.5 | 9.71±2.12 |
| 2h | 988±108 | 23.8±6.83 |
| 4h | 925±42.0 | 54.7±16.0 |
| Day 1 | 462±14.7 | 348±21.1 |
| Day 3 | 294±11.9 | 292±10.2 |
| Day 5 | 213±12.5 | 262±13.9 |
| Day 8 | 136±19.0 | 190±11.1 |
| Day 11 | 96.5±7.08 | 123±17.5 |
| Day 14 | 81.5±14.2 | 95.1±14.5 |
| Day 21 | 22.1±4.69 | 29.1±2.77 |
| Day 25 | 15.6±3.29 | 17.5±1.62 |

**Table 8. Pharmacokinetic properties of fusion protein 1 in SD rats**

| Pharmacokinetic parameter | Intravenous injection | Subcutaneous injection |
|---|---|---|
| Dose (nmol/kg) | 30 | 30 |
| t1/2 (days) | 4.49 | 4.45 |
| Tₘₐₓ (days) | 0.0104 | 1 |
| Cₘₐₓ (nmol/L) | 1455 | 348 |
| AUC _{0-inf_obs} (nmol/L*d) | 3700 | 3481 |
| MRT _{0-inf_obs} (d) | 6.44 | 8.21 |
| V_{z_obs} (mL/kg) | 52.6 | 55.3 |
| Cl_{_obs} (mL/kg/d) | 8.10 | 8.62 |
| %F | NA | 94.1 |

The results show that fusion protein 1 exhibited ultra-long *in-vivo* pharmacokinetic properties after being administered via a single intravenous/subcutaneous injection in normal rats, with a half-life of about 4.5 days, indicating that the fusion protein may have a lower frequency of administration and a longer administration interval in clinical practice.

### Example 7. Pharmacodynamic Study of Human Insulin Analog-Fc Fusion Proteins in Type I Diabetic Rat model

In this example, streptozotocin (STZ)-induced rats were used as a type I diabetic animal model to examine the hypoglycemic effects and duration after administration of some of the human insulin analog-Fc fusion proteins via a single subcutaneous injection, so as to comprehensively assess the hypoglycemic activity and *in-vivo* pharmacokinetic properties.

Experimental method: Male SD rats (purchased from Vital River) weighing about 350 g and aged 8 weeks were selected and then acclimated for one week. A citric acid/sodium citrate buffer with a pH range of 4.2-4.5 was prepared, and STZ was dissolved in this buffer to a concentration of 1% (w/v). The STZ solution was then injected intraperitoneally into the rats based on the actual fasting body weight at a dose of 60 mg/kg per rat. On the third day after the STZ injection, the fasting blood glucose of the rats was tested. If the measured blood glucose level was higher than 15 mM, the modeling was considered successful.

Each human insulin analog-Fc fusion protein was prepared to the corresponding concentration using 1× PBS buffer at pH 7.4. Before administration, the blood glucose level of each type I diabetic rat was tested, and rats with blood glucose values between 20-30 mM were selected and randomly grouped, ensuring that the average blood glucose and standard deviation of each group of rats (3 or 6 rats) were comparable. After the grouping was completed, each group of rats was given the corresponding human insulin analog-Fc fusion protein via subcutaneous injection based on the body weight. After administration, the rats were allowed to eat and drink water normally, and the blood glucose values of the animals were continuously monitored at 1 h, 2 h, 4 h, 24 h, 48 h, etc. after administration, until the blood glucose levels returned to pre-administration blood glucose levels. A corresponding graph of blood glucose values over time was then plotted.

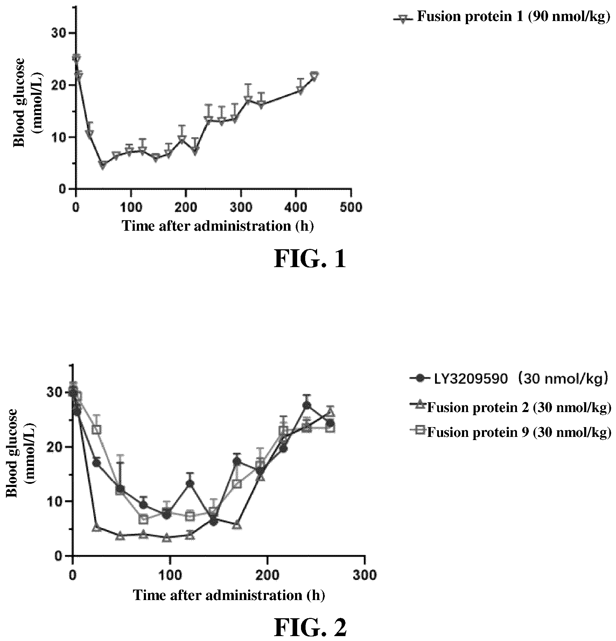

In the STZ-induced type I diabetic rat model, the time-dependent changes in blood glucose concentration after a single subcutaneous injection of fusion protein 1 (administered at a dose of 90 nmol/kg) are shown in FIG. 1; the time-dependent changes in blood glucose concentration for LY3209590, fusion protein 2, and fusion protein 9 (each administered at a dose of 30 nmol/kg) are shown in FIG. 2. The results show that fusion protein 1, fusion protein 2, and fusion protein 9 all exhibited long-time hypoglycemic effects, wherein the hypoglycemic effect after the single subcutaneous injection of fusion protein 1 could last for at least 300 h.

### Example 8. Pharmacodynamic Study of Human Insulin Analog-Fc Fusion Proteins in db/db Type II Diabetic Mouse Model

In this example, a db/db type II diabetic mouse model was used to examine the improvement in glycated hemoglobin after administration of some of the human insulin analog-Fc fusion proteins via multiple subcutaneous injections, so as to comprehensively assess the hypoglycemic activity and *in-vivo* pharmacokinetic properties.

Experimental method: 50 db/db mice were purchased and acclimated for 5 days, and then animals with random blood glucose levels higher than 20 mmol/L were selected and further grouped for the experiment. The experiment was performed using a total of 40 mice, which were divided into 4 groups of 10: a model control group (vehicle control group, components: 0.08 M histidine, 70 g/L sucrose, and 0.4 g/L polysorbate 80), a fusion protein 1-low dose group, a fusion protein 1-medium dose group, and a fusion protein 1-high dose group. Starting from the first day after the grouping, the administration was performed by subcutaneous injection once a week for a total of 4 times. At the endpoint of the experiment, glycated hemoglobin was detected in all mice. The procedures for the detection were as follows: 0.4 mL of blood was taken from the main abdominal vein of each animal, then injected into an EDTA anticoagulation tube, and directly detected for glycated hemoglobin on a machine. The results are shown in Table 9.

**Table 9. Results for glycated hemoglobin in animals (%)**

| **Group** | **Administration regimen** | **RWD%** |
|---|---|---|
| Model control group | Vehicle | 11.27±0.87 |
| Fusion protein 1-low dose group | Fusion protein 1, 20 nmol/kg | 11.01±0.86 |
| Fusion protein 1-medium dose group | Fusion protein 1, 40 nmol/kg | 10.58±0.44* |
| Fusion protein 1-high dose group | Fusion protein 1, 80 nmol/kg | 10.41±0.45* |

| | | |
|---|---|---|
| Note: The results are presented as mean ± SD; "*" indicates p ≤ 0.05 compared to the model control group. | | |

According to the experimental results, the content of glycated hemoglobin in the model control group was 11.27±0.87%, the contents of glycated hemoglobin in the low, medium and high dose groups of fusion protein 1 were 11.01±0.86%, 10.58±0.44% (p = 0.0481), and 10.41±0.45% (p = 0.0171), respectively; the medium dose group and the high dose group showed statistical differences compared to the model control group, and there was a certain dose-effect relationship among the three dose groups. The above results suggest that the medium and high dose groups of fusion protein 1 could significantly reduce the content of glycated hemoglobin in the db/db mice, while the effect of the low dose group was not significant, and there was a certain dose-effect relationship among the three dose groups.

### Example 9. Assay on Pharmacokinetic Properties of Human Insulin Analog-Fc Fusion Proteins in Bama Pigs

In this example, Bama pigs were used as an animal model to study the pharmacokinetic properties of fusion protein 1 and LY3209590 after a single subcutaneous injection in Bama pigs.

Experimental method: Male Bama pigs (Yibin Hengshu Bio-Technology Co., Ltd.) weighing 13-18 kg and aged 4-6 months were selected. Fusion protein 1 and LY3209590 were prepared using a vehicle (components: 80 mM histidine, 70 g/L sucrose, and 0.4 g/L Tween 80) and 1× PBS buffer, respectively, and then injected subcutaneously into the animals at doses of 5 nmol/kg and 2 nmol/kg, respectively. About 1 mL of venous blood was collected from each animal at 1 h, 4 h, 8 h, 24 h, 48 h, 72 h, 96 h, 120 h, 144 h and 168 h after administration, and the blood samples were not anticoagulated. The whole blood samples were temporarily stored in an ice box and then centrifuged at 2 to 8 °C at about 1800 ×g for 10 min. 2 tubes of serum were isolated (with one tube containing about 100 µL, and the remaining serum stored in another tube) and then stored at a temperature below -66 °C for the analysis of *in-vivo* pharmacokinetic properties. The analysis of *in-vivo* pharmacokinetic properties was performed using a method similar to that described in Example 6, with the specific pharmacokinetic parameters shown in Table 10.

**Table 10. Pharmacokinetic properties of fusion protein 1 and LY3209590 in Bama pigs**

| Compound | Fusion protein 1 | LY3209590 |
|---|---|---|
| Dose (nmol/kg) | 5 | 2 |
| t1/2 (h) | 120 | 84 |
| Tₘₐₓ (h) | 19 | 25 |
| Cₘₐₓ (ng/mL) | 2900 | 1530 |
| AUC _{0-inf_obs} (h*ng/mL) | 511000 | 201000 |
| MRT _{0-inf_obs} (h) | 180 | 130 |

The results show that fusion protein 1 exhibited ultra-long *in-vivo* pharmacokinetic properties after being administered via a single subcutaneous injection in Bama pigs. At the doses shown in Table 10, the half-lives (t1/2) of the drugs did not change significantly with the doses. Compared to LY3209590, the fusion protein 1 had a significantly prolonged half-life of about 120 h, indicating that this fusion protein has a lower frequency of administration and a longer administration interval in clinical practice.

The present disclosure provides novel fusion proteins of human insulin analogs and the IgG Fc region, which can be used to treat metabolic diseases such as type I diabetes or type II diabetes. The fusion proteins exhibited ultra-long *in-vivo* pharmacokinetic properties, showed good and sustained hypoglycemic effects in the STZ-induced type I diabetic rat model and the db/db type II diabetic mouse model, had excellent physical/chemical stability and high expression yields, and demonstrated significantly lower *in-vitro* cell proliferation-inducing activity compared to natural human insulin, suggesting the fusion proteins have potentially good safety and lower medication risk and thus can meet the clinical demand for ultra-long-acting insulin products.

## Claims

1. A fusion protein, comprising:
an insulin analog having the following general formula from the N-terminus to the C-terminus: B₁-L₁-A₁,
wherein
B₁ is an insulin B-chain analog comprising the following amino acid sequence:
FVX₁QHLCGX₂HLVEALX₃X₄VCGERGFX₅Y (SEQ ID NO: 1), wherein
X₁ is selected from the group consisting of N, G, and K,
X₂ is selected from the group consisting of S and E,
X₃ is selected from the group consisting of Y, H, and E,
X₄ is selected from the group consisting of H, R, L, and E, and
X₅ is selected from the group consisting of F and H;
A₁ is an insulin A-chain analog comprising the following amino acid sequence:
GIVEQCCZ₁SICSLZ₂QLENYCZ₃ (SEQ ID NO: 2), wherein
Z₁ is selected from the group consisting of T, H, and E,
Z₂ is selected from the group consisting of Y, D, S, and E, and
Z₃ is selected from the group consisting of G and N;
L₁ is a linker, preferably a GS linker and/or a linker having at least five G, more preferably a linker with the amino acid sequence set forth in SEQ ID NO: 3 or 52.

2. The fusion protein according to claim 1, wherein
X₁ is selected from the group consisting of N and K, X₂ is selected from the group consisting of S and E, X₅ is selected from the group consisting of Y and H, X₄ is selected from L, and X₅ is selected from H;
Z₁ is selected from the group consisting of T, H, and E, Z₂ is selected from E, and Z₃ is selected from the group consisting of G and N.

3. The fusion protein according to claim 1 or 2, wherein
the insulin B-chain analog comprises or is selected from the amino acid sequence set forth in any one of SEQ ID NOs: 31-36, and
the insulin A-chain analog comprises or is selected from the amino acid sequence set forth in any one of SEQ ID NOs: 37-42;
preferably, the fusion protein comprises the amino acid sequence set forth in any one of SEQ ID NOs: 4-9.

4. The fusion protein according to any one of claims 1-3, further comprising C₁, wherein the C₁ is selected from at least one of the following: an Fc region of an immunoglobulin, HSA, and an HSA-binding domain;
preferably, the C₁ is selected from an Fc region of IgG;
more preferably, the C₁ is selected from the group consisting of Fc regions of human IgG1, IgG2 and IgG4.

5. The fusion protein according to claim 4, wherein the C₁ is located at the N-terminus or C-terminus of the insulin analog, preferably at the C-terminus of the insulin analog.

6. The fusion protein according to any one of claims 4-5, wherein the C₁ comprises or is the amino acid sequence set forth in any one of SEQ ID NOs: 17, 18, and 48-51, or an amino acid sequence having at least 90% identity thereto.

7. The fusion protein according to any one of claims 4-6, further comprising a linker L₂, wherein C₁ forms the fusion protein with B₁-L₁-A₁ via L₂;
preferably, the L₂ is selected from any one of the following: (GₘQᵢ)ₙ, (GₘAᵢ)ₙ, (GₘQ)ₙ, (GₘA)ₙ, and (PGPQ)ₛ, wherein
each m is independently selected from the group consisting of integers of 1-10,
each n is independently selected from the group consisting of integers of 1-10, and
each i is independently selected from the group consisting of 0-4;
s is selected from the group consisting of integers of 1-10;
more preferably, the L₂ comprises or is the amino acid sequence set forth in any one of SEQ ID NOs: 10-16.

8. The fusion protein according to any one of claims 4-7, comprising or being the amino acid sequence set forth in any one of SEQ ID NOs: 19-27, or an amino acid sequence having at least 90% sequence identity thereto.

9. The fusion protein according to any one of claims 1-8, being a dimer, preferably a homodimer.

10. The fusion protein according to any one of claims 1-9, wherein the insulin analog is an insulin receptor agonist with insulin receptor agonistic activity.

11. An insulin analog, comprising an insulin B-chain analog and an insulin A-chain analog, wherein
the insulin B-chain analog comprises the following amino acid sequence:
FVX₁QHLCGX₂HLVEALX₃X₄VCGERGFX₅Y (SEQ ID NO: 1), wherein X₁ is selected from the group consisting of N, G, and K, X₂ is selected from the group consisting of S and E, X₃ is selected from the group consisting of Y, H, and E, X₄ is selected from the group consisting of H, R, L, and E, and X₅ is selected from the group consisting of F and H;
the insulin A-chain analog comprises the following amino acid sequence: GIVEQCCZ₁SICSLZ₂QLENYCZ₃ (SEQ ID NO: 2), wherein Z₁ is selected from the group consisting of T, H, and E, Z₂ is selected from the group consisting of Y, D, S, and E, and Z₃ is selected from the group consisting of G and N;
preferably, X₁ is selected from the group consisting of N and K, X₂ is selected from the group consisting of S and E, X₃ is selected from the group consisting of Y and H, X₄ is selected from L, and X₅ is selected from H; Z₁ is selected from the group consisting of T, H, and E, Z₂ is selected from E, and Z₃ is selected from the group consisting of G and N.

12. The insulin analog according to claim 11, wherein
the insulin B-chain analog comprises or is selected from the amino acid sequence set forth in any one of SEQ ID NOs: 31-36, and
the insulin A-chain analog comprises or is selected from the amino acid sequence set forth in any one of SEQ ID NOs: 37-42;
preferably,
the insulin B-chain analog and the insulin A-chain analog respectively comprise or are selected from the amino acid sequences set forth in any one of the following groups:
SEQ ID NOs: 31 and 37;
SEQ ID NOs: 32 and 38;
SEQ ID NOs: 33 and 39;
SEQ ID NOs: 34 and 40; and
SEQ ID NOs: 36 and 42.

13. The insulin analog according to claim 11 or 12, wherein a disulfide bond is formed between the insulin A-chain analog and the insulin B-chain analog.

14. A glucose-responsive insulin, comprising the insulin analog according to any one of claims 11-13.

15. A polynucleotide, encoding the fusion protein according to any one of claims 1-10, or the insulin analog according to any one of claims 11-13, or the glucose-responsive insulin according to claim 14.

16. A host cell, comprising the polynucleotide according to claim 15.

17. A method for preparing a fusion protein or an insulin analog, comprising:
culturing the host cell according to claim 16;
collecting the fusion protein according to any one of claims 1-10 or the insulin analog according to any one of claims 11-13; and
optionally, purifying and/or modifying the fusion protein or the insulin analog.

18. A pharmaceutical composition, comprising the fusion protein according to any one of claims 1-10, the insulin analog according to any one of claims 11-13, or the glucose-responsive insulin according to claim 14; and one or more pharmaceutically acceptable carriers, diluents or excipients.

19. A method for treating diabetes and a complication thereof or lowering blood glucose, comprising administering to a subject in need thereof a therapeutically effective amount of the fusion protein according to any one of claims 1-10, the insulin analog according to any one of claims 11-13, the glucose-responsive insulin according to claim 14, or the pharmaceutical composition according to claim 18, wherein
preferably, the diabetes and the complication thereof are selected from the group consisting of: type I diabetes, type II diabetes, and complications thereof;
more preferably, the complication is selected from the group consisting of diabetes-related heart disease, stroke, retinopathy, neuropathy and kidney disease.
